# EUROPEAN PATENT SPECIFICATION

(11) **EP 1 724 019 B1**
(45) Date of publication and mention of the grant of the patent: **26.11.2008**
(21) Application number: 06252562.1
(22) Date of filing: 17.05.2006
(51) Int. Cl.: B01L 3/00

(54) **Receiver plate with multiple cross-sections**
Aufnahmeplatte mit mehreren Querschnitten
Récepteur plaque avec sections transversales multiples

(30) Priority: 19.05.2005 US 132996
(43) Date of publication of application: 22.11.2006
(73) Proprietor: MILLIPORE CORPORATION, Billerica, Massachusetts 01821-3405 (US)
(72) Inventor: Scott, Christopher A., Westford, Massachusetts 01886 (US); Foley, Brian, Westford, Massachusetts 01886 (US)
(74) Representative: Greenwood, John David

(56) References cited:
- EP-A- 1 524 033
- US-A- 5 650 323
- US-A1- 2003 215 956

## Description

### BACKGROUND OF THE INVENTION

The bioavailability of a drug is affected by a number of factors including its ability to be absorbed into the blood stream through the cells lining the intestines. There are a number of different *in vitro* assay options available to predict the gastrointestinal absorption property of drugs including a permeability assay, and a method known as PAMPA (Parallel Artificial Membrane Permeability Assay), which uses a lipid filled membrane to simulate the lipid bilayer of various cell types, including intestinal epithelium. These non-cell based permeability assays are automation compatible, relatively fast (4-24 hours), inexpensive, and straightforward. They are being used with increasing frequency to determine the passive, transcellular permeability properties of potential drug compounds. The majority of drugs enter the blood stream by passive diffusion through the intestinal epithelium. Consequently, permeability assays that measure passive transport through lipophilic barriers correlate with human drug absorption values from published methods.

Assays that predict passive absorption of orally administered drugs have become increasingly important in the drug discovery process. The ability of a molecule to be orally absorbed is one of the most important aspects in deciding whether the molecule is a potential lead candidate for development. Cell-based assays, like those using Caco-2 cells, are commonly used as a model for drug absorption; however, the technique is labor intensive and is often situated late in the drug discovery process. Assays described by Kansy and Faller have addressed these issues by providing rapid, low cost and automation friendly methods to measure a compound's passive permeability. Both permeability and PAMPA assays use artificial membranes to model the passive transport properties of the cell membrane. Other researchers have presented variations on Kansy's method, in some cases, improving on the correlation with a particular target (e.g., blood-brain barrier) or class of molecules. In general, the original assay has remained the same.

The devices used to carry out permeability assays include a filter plate containing one or more wells with a membrane barrier fixed to the bottom of each well, and a receiver plate configured to receive the filter plate in a nested relationship. Reagents and buffers are placed with the filter wells and the receiver wells at specific volume ratios so that accurate drug transport data can be analyzed. It is desirable to have the filter plate wells with membrane inserted into the receiver plate wells so that the media in the receiver plate wells will be at or close to equal level with the media in the filter wells. This creates hydrostatic equilibrium and minimized pressure differentials, which can cause uncontrolled or forced diffusion through the membrane. At a minimum however, the membrane must remain in contact with the liquid in the receiver plate during the experiment, including during incubation, shaking, and mixing. Cell culture assays (e.g., Caco-2) and non-cell based screening assays (e.g., PAMPA) are described in this manner. These devices also have non-cell based applications, which offer higher throughput compared to Caco assays, and require larger membrane areas to help achieve this.

Analysis is performed by reading directly in a transport assembly with UV or visual readers. It is therefore desirable to have a receiver plate that allows UV and visual light transmission. The protocol may also require shaking or other means of agitating the media, as well as extended incubation at room temperature. Handling of the device can be done manually or with automated plate handlers. In the latter case, the device needs to be compatible with the ANSI/SBS Microplate Standards (incorporated herein by reference) which apply mainly to the size, shape, and profile of the outer walls of the plate. These standards also restrict the well array by standardizing the distance between well centers and the location of the array relative to the outside of the plate.

US-A-5,650,323 discloses laboratory research tool for conducting a variety of procedures including cell and tissue culture techniques includes a multi-well cluster plate, a filter plate that has one or more filter wells that extend into the wells of the cluster plate, a cover and a reservoir that can be used with auxiliary equipment designed for use with standard 96-well format cluster plates. The system allows multiple tissue samples to be grown and manipulated simultaneously

US-A-2003/0215956 discloses multi-well plates and column arrays in which samples (e.g., cell lysates containing nucleic acids of interest, such as RNA) can be analyzed and/or processed. In one embodiment, the microfiltration arrangement is a multilayer structure, including (i) a column plate having an array of minicolumns into which samples can be placed, (ii) a discrete filter element disposed in each mini-column, (iii) a drip-director plate having a corresponding array of drip directors through which nitrate may egress, and (iv) a receiving-well plate having a corresponding array of receiving wells into which filtrate can flow. The invention provides multi-well microfiltration arrangements that are relatively simple to manufacture and that overcome many of the problems associated with the prior arrangements relating to (i) cross-contamination due to wicking across a common filter sheet or (ii) individual filter elements entrapping sample constituents within substantial dead volumes. Further, the invention provides multi-well microfiltration arrangements that adequately support discrete filter elements disposed in the wells without creating substantial preferential flow. Additionally, the invention provides multi-well microfiltration arrangements that avoid cross-contamination due to aerosol formation, pendent drops and/or splattering. Other disclosed features of the invention provide for the automated covering or heat-sealing of filtrate samples separately collected in an array of wells.

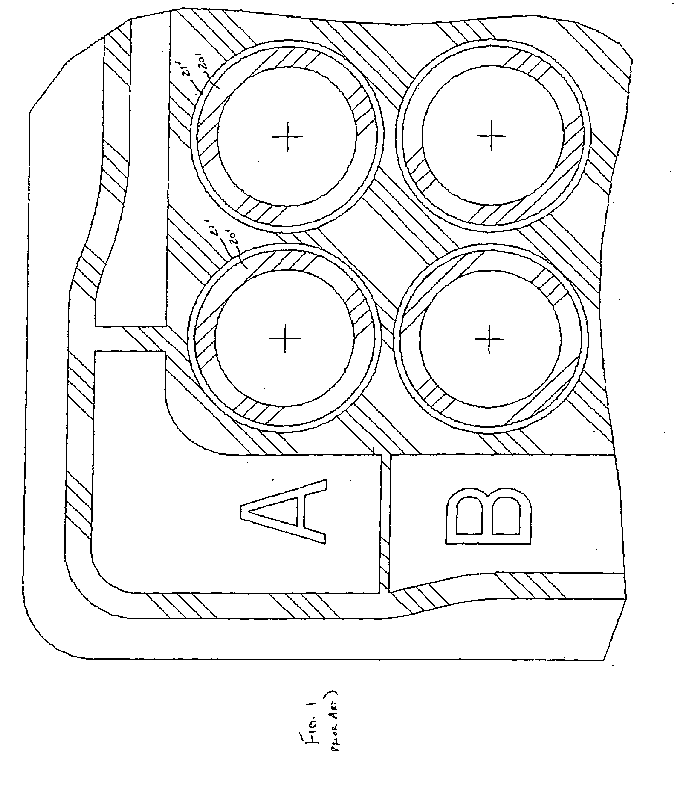

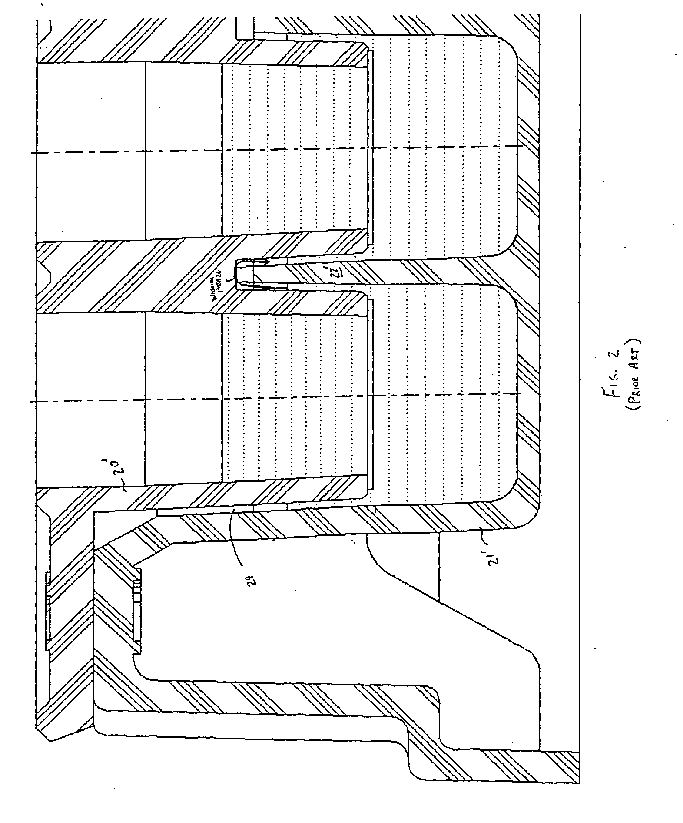

Figures 1 and 2 of the present application show a prior-known filter plate which has the features of the preamble of claim 1. The present invention is characterized by the features of the characterizing portion of claim 1. Optional features are recited in the dependent claims.

Conventional receiver plates used in non-cell based PAMPA type assays include opaque acceptor plates and clear polystyrene receiver plates. Capillary wicking, cross contamination, volume control, evaporation, automation compatibility, and liquid recovery are problematic in these devices, however. The primary cause of cross contamination is the wicking of liquid in the small gap between each filter well and receiver well when the two plates are nested together, especially during incubation and shaking of the device. In conventional devices each receiver plate well has a circular cross section and thus forms a uniform capillary gap with a corresponding well of the concentrically nested filter plate, allowing for the wicking and cross contamination to occur. With these conventional devices, the cross-section is also uniform from the top to the bottom of the well, which increases the volume in the lower section of the well located under the membrane. Also with these conventional devices, the uniform capillary gap in the upper section of the well can hold only a minimum volume of media, and therefore when the device is assembled, there is a greater chance of displacing liquid out of the well, which leads to cross contamination. In the conventional devices, there are no features to assist in automated assembly and disassembly of the filter plate with the receiver plate. In conventional devices, the filter plate nests in the receiver plate such that there is a gap between the two, thus creating open paths to the atmosphere for evaporation of media from the receiver wells.

It therefore would be desirable to provide a receiver plate that reduces or eliminates capillary wicking and cross contamination.

It further would be desirable to provide a receiver plate that readily accommodates visual readers.

It further would be desirable to provide a receiver plate that minimizes the media volume requirements in the receiver plate.

It further would be desirable to provide a receiver plate that can handle a wider range of receiver volumes such that the membrane remains in liquid contact and the media does not displace out of the wells when the device is assembled.

It further would be desirable to provide a receiver plate that has features to assist in the automated assembly and disassembly of the filter plate.

It further would be desirable to provide a receiver plate that will nest such that each filter well is centered within each receiver well with minimal variation during the course of the experiment.

It still further would be desirable to provide a receiver plate that minimizes the effects of evaporation of media from the wells during non-humidified incubation.

### SUMMARY OF THE INVENTION

The problems of the prior art have been overcome by the present invention, which provides a multi-well assembly including a filter plate and a receiver plate. Each plate includes a plurality of wells, which, when the filter plate is placed in a nesting relationship with the receiver plate, each filter plate well has a corresponding receiver plate well into which it extends in nesting relationship. The receiver plate wells are of a non-uniform cross section along the height of the well. The cross-section of the upper portion of the receiver plate well is chosen to increase the gap between the outer walls of the filter plate wells and the inner walls of a corresponding receiver plate well when the receiver plate and filter plate are in a nesting relationship. This cross section creates a non-uniform gap such that the increased gap size reduces wicking and cross-contamination as well as increases the volume around the filter well to accommodate larger media volume variations. The lower portion of the receiver plate well has a reduced cross section compared to the upper portion, thus forming a non-uniform cross-section along the well height. This reduced volume lower section reduces the media required for the experiment. The cross-section of the receiver plate wells transitions from a substantially square cross-section to a substantially round cross-section at a point at or below where the membrane on the filter plate well would be positioned when the filter plate is in nesting relationship with the receiver plate. The square cross-section also provides larger pathways for air to escape during assembly of the device. A square cross section maximizes the useable space between neighboring wells given a circular filter well and the limitations of ANSI/SBS restrictions on well spacing. A multi-section well of maximum cross-section in an upper region and a minimized cross-section in a lower region, with a gradual transition between the regions, is thus provided.

The multi-well assembly of the present invention also improves the repeatability of positioning the filter plate and receiver plate in proper nesting relationship, so that the filter wells are not eccentric with the receiver wells. The present invention also provides a means to improve automated assembly and disassembly by means of a lead-in feature. In addition, evaporation of media from the receiver wells is reduced by providing a flat surface-to-surface contact between the filter plate and receiver plate.

### BRIEF DESCRIPTION OF THE DRAWINGS

Figure 1 is a cross-sectional top view showings wells of a conventional filter plate nested in a receiver plate;
Figure 2 is a cross-sectional side view showings wells of a conventional filter plate nested in a receiver plate;
Figure 3 is a cross-sectional side view showing a portion of a filter plate in nesting relationship with a receiver plate in accordance with the present invention;
Figure 4 is a perspective view showing a portion of a filter plate in nesting relationship with a receiver plate in accordance with the present invention;
Figure 5 is a perspective view of a receiver plate in accordance with the present invention;
Figure 6 is a perspective view of a portion of a filter plate in nesting relationship with a receiver plate showing filter plate support ribs and a positioning rib in accordance with the present invention; and
Figure 7 is a perspective view of a portion of a filter plate nested in a receiver plate and showing a position rib in accordance with the present invention.

### DETAILED DESCRIPTION OF THE INVENTION

Turning first to Figures 1 and 2, there is shown conventional filter plate wells 20' nested in conventional receiver plate wells 21'. The filter plate wells 20' have a uniform circular cross-section, and the receiver plate wells 21' have a uniform circular cross-section as well. The outside diameter of the filter plate wells 20' is slightly smaller than the inside diameter of the receiver plate wells 21', enabling the filter plate wells 20' to be nested within the receiver plate wells as seen in Figure 2. A small capillary gap 24 is formed between the outer walls of the filter plate wells 20' and the inner walls of the corresponding receiver plate wells 21', as well as between the inner walls of the filter plate wells 20' and the wall 22'separating receiver plate wells 21'. This gap allows for displacement and wicking of fluid and results in cross-contamination, as fluid from one receiver plate well can travel in the gap and contaminate fluid in another well, as shown by the wicking path 26 in Figure 2.

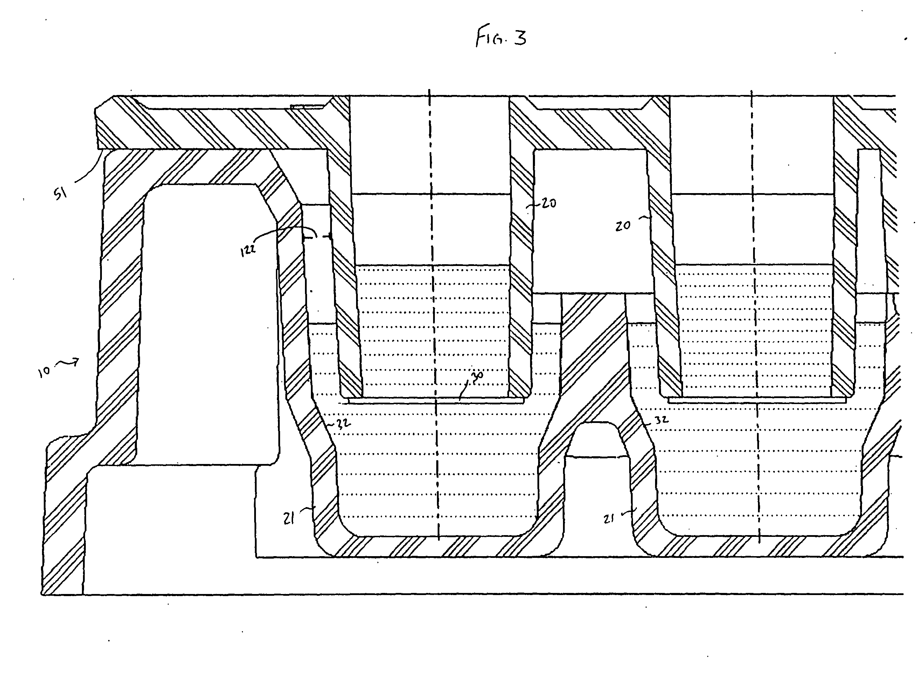

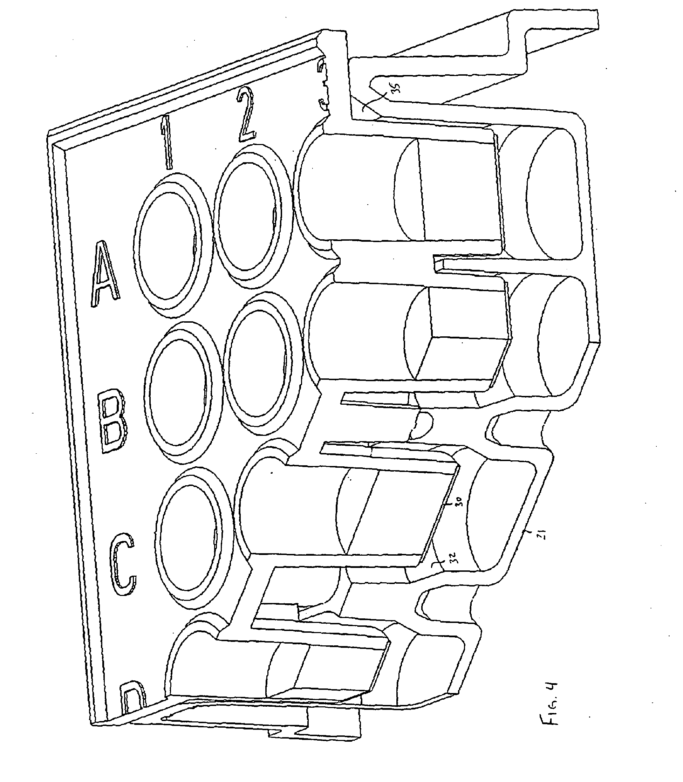

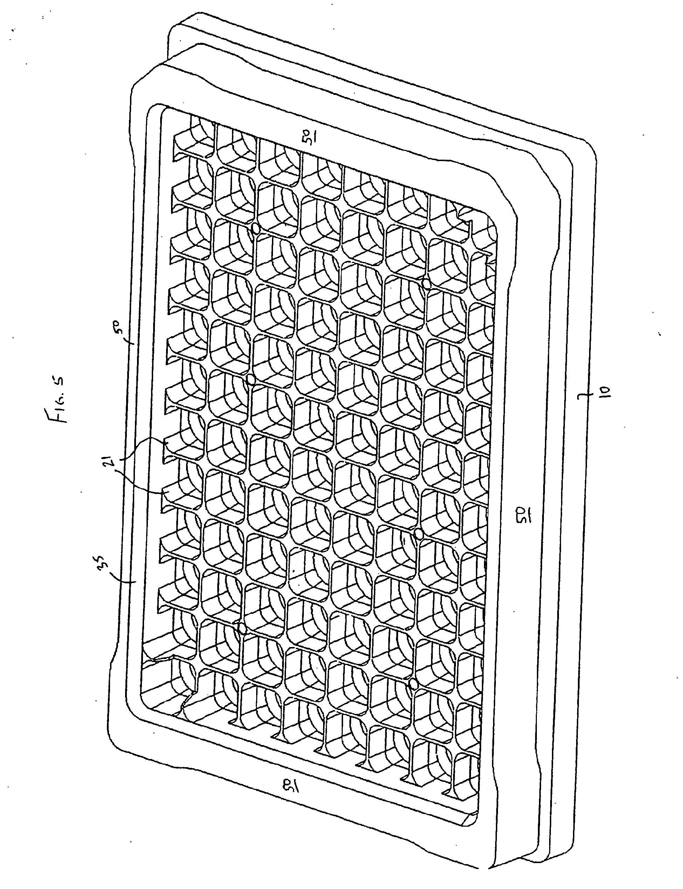

Figures 3 and 4 illustrate a preferred embodiment of the present invention that increases the gap between the outer walls of the filter plate wells and inner walls of the receiver plate well in order to reduce or eliminate wicking and cross contamination between or among wells and to reduce the chance of displacing liquid out of the well. In the embodiment shown, the diameter in the portion of each receiver plate well 21 that receives a filter plate well 20 is increased, so that the gap 122 between the inner walls of each receiver plate well 21 and the outer walls of a corresponding filter plate well 20 is increased, thereby inhibiting capillary action and reducing or eliminating wicking of fluid in this gap. This portion of each receiver plate well has a substantially square cross-section, as seen in Figure 5. This increased diameter region of the well also provides a pathway for air to escape when the filter plate is placed in nesting relationship with the receiver plate. Any air bubbles that otherwise would become trapped underneath the membrane during the assembly of the plates now can travel out the gap between the filter plate well and the receiver plate well.

The upper region of the receiver plate is in a square cross-section and there is a suitable gap between the outer wall of a nested filter plate well 20 and the inner wall of a receiver plate well 21 at the four corners of the square with a maximum of 0.099 cm (0.039 inches), depending on the corner radius chosen, with a minimum gap of about 0.0254 cm (0.010 inches) at the four side walls. The minimum gap is dictated by the ANSI/SBS array spacing standard and the outside diameter of the filter plate well 21'.

Since wicking of fluid is not an issue in the region of each receiver well below where each filter plate well nests when in the assembled condition (e.g., in the region of each receiver plate well that is below the effective area of the membrane 30 of each filter plate well), the diameter and therefore the volume of this region can be smaller than the region that receives and accommodate each filter plate well. According to the present invention, each receiver plate well 21 transitions from a larger diameter region in the area that receives a filter plate well 20 to a smaller diameter region in the area that is below where each filter plate well 20 nests. This region of each receiver plate well 21 is circular in cross-section which improves liquid recovery and controls the amount of media volume for the experiment. More particularly, were the square cross-section continued from the region that receives the filter plate well to the region below where the filter plate well nests, un-recovered media would become trapped in the well corners, especially during automated liquid removal in which a pipette is extracting liquid from a single location and tipping the plate is not possible. Since it is only necessary to have media directly under the effective membrane area, the diameter of the lower region of the receiver plate well can be substantially the same as the outer dimensions of the filter well 20, and preferably the same as the effective membrane diameter so that fluid can transfer between each filter well and corresponding receiver well, through the membrane, without obstruction, to this region of the receiver well. Preferably no region of the receiver plate well is less than the effective membrane area, so that the entire membrane surface remains visible to plate readers when viewed from the bottom of the plate.

The transition from the larger diameter region to the smaller diameter region is preferably uniform in order to reduce hold up or un-recovered media. In the embodiment shown, the transition results in angled wall sections 32 when cross sections are taken through corners of the square well, as shown in Figure 3. This angled section will vary from zero degrees (as measured from a vertical axis) for sections taken through the side walls (as shown in Figure 2) to a maximum angle which is dictated by the corner radius and the height of the transition. The shape of the upper and lower sections and the height of translation will determine the maximum angles that are produced. For proper drainage, it is desirable to have angles less than 70 degrees when measured from vertical.

Proper and reproducible placement of the filter plate wells within the receiver plate wells is important to avoid cross contamination, as eccentric nesting of the filter plate wells in the receiver plate wells can cause the gap between the wells to vary and allow wicking. Also, the well location needs to be properly maintained through the experiment during manual and automated handling, mixing, and shaking to prevent liquid sloshing, spilling, and wicking. Proper placement, particularly during automation, can be enhanced in accordance with one embodiment of the present invention by providing a chamfer 35 along the outside perimeter of the array of wells in the receiver plate 10. The chamfer functions to guide the outside edges of filter plate wells 20 into proper nesting relationship with the receiver plate wells 21. By locating the chamfer around the perimeter of the well array, various configurations of filter plates can be guided into place, even where the filter late has a skirt that would interfere with such a guide were it positioned about the outside edge of the receiver plate rather than about the periphery of the well array. Preferably the chamfer is formed at a 45° angle, sloping toward the wells as seen in Figure 5.

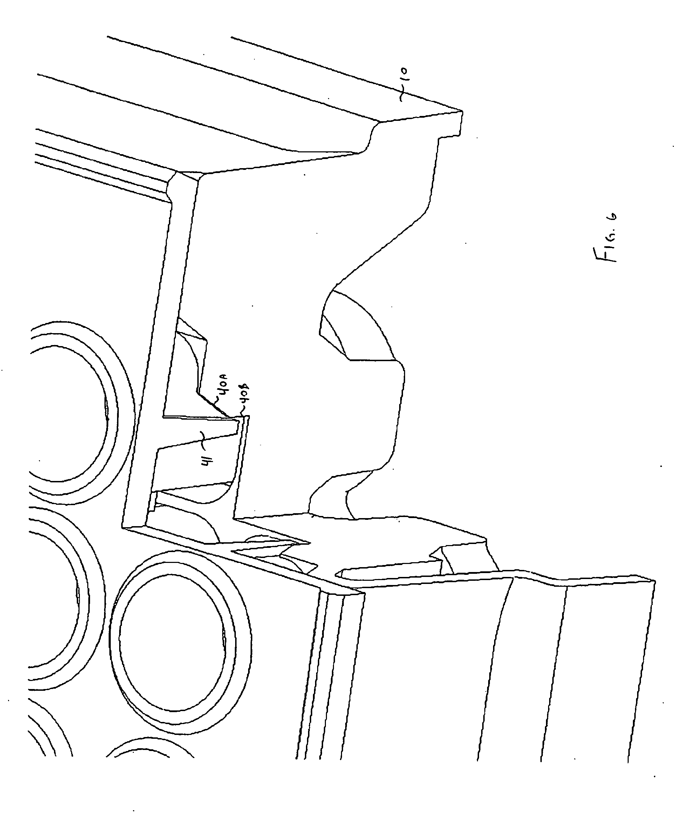

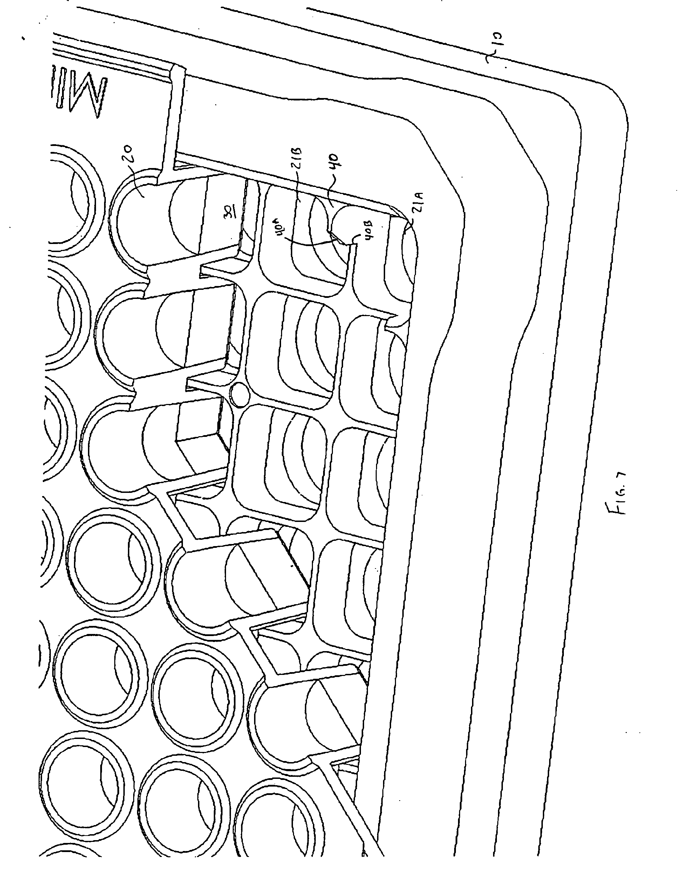

To further guide the assembly of the filter plate and receiver plate, positioning ribs or posts 40 can be provided in one or more, preferably at least two, wells of the receiver plate that mate with corresponding well support ribs or posts 41 in corresponding filter plate wells. The positioning ribs also provide a means of keeping the filter plate from moving or shifting during handling, mixing, and shaking. As best seen in Figure 7, a positioning rib 40 is provided between a corner well 21 A and an adjacent well 21 B in the receiver plate 10. Preferably the rib 40 has a flat top that extends towards the well array and is slightly lower than the top face of the receiver plate. The rib 40 terminates in a sidewall that includes a chamfered region 40A, preferably angled at about 45°, and a straight or perpendicular portion 40B perpendicular to the top of the well wall. The chamfered lead-in and tapered mating face of the rib 40 guide a corresponding support rib 41 (Figure 6) on a corresponding well of the filter plate. Preferably the support ribs 41 are tapered, narrowing towards their free ends. Positioning ribs 40 are only necessary at two points in the plate to control translation in two directions and rotation about the vertical axis. Although the positioning ribs 40 are preferably located at opposite corner wells, it is within the scope of the present invention to provide them at any point within the plate, including the outside well walls or the gap between well walls (in which case the rib would fit in between the filter plate wells with a chamfer lead in and tapered wall on each side). The rib or pin features could also use the outside wall of the filter plate 20' to provide the means of location.

In order to reduce evaporation of media from the receiver wells, particularly from the peripheral receiver wells, the filter and receiver plates preferably are configured so that there is a flat surface-to-surface contact area between the plates to seal the wells. Thus, the area 50 that is peripheral to the chamfered lead-in 35 of the receiver plate is flat or planar (Figure 5), as is the corresponding area 51 of the filter plate that sits on area 50 (Figure 3), the effectively creating a face seal when the filter plate is in nesting relationship with the receiver plate. This allows the filter wells to hang in the receiver wells, and eliminates communication with the outside environment. There are other means for ensuring a region of intimate contact between the filter plate and receiver plate with no air gap and minimum contact with the environment. One such method is a raised bead around the periphery of the receiver plate. This raised bead could be an overmolded elastomeric material, thus acting as a gasket type seal.

## Claims

1. A multi-well device comprising a filter plate having a plurality of filter wells (20), each of which includes a membrane (30) located at the bottom of the well, and a receiver plate having plurality of receiver wells (21) each having a bottom, said receiver plate adapted to receive said filter plate, in nesting relationship whereby each of said filter wells (20) extends into a respective receiver well (21) to a position spaced from said receiver well bottom, thereby defining in each said receiver well a first region being a filter well occupied region and a second region being a region unoccupied by a filter well, said first region having a cross-section larger than said second region, **characterized in that** each of said receiver wells has a region (32) of non-uniform cross-section providing a transition between the cross-section of said first region of each receiver well (21) and the cross-section of said second region of said receiver well (21), said receiver well (21) transitions from said first region to said second region at a point at or below the location of each said fitter plate well membrane (30) when each said filter plate well (20) is in nesting relationship with a corresponding receiver plate well (21), and said first region has a substantially square cross-section and said second region has a substantially circular cross-section.

2. A multi-well device as claimed in claim 1, said receiver plate having, a chamfer (35) formed along the periphery of said array of receiver wells (21).

3. The multi-weli device of claim 1, wherein each said membrane (30) has an area effective to carry out filtration and wherein the second region of each said receiver well (21) is configured such that fluid can transfer unobstructed from said area of each said membrane (30) to each said second region.

4. The multi-well device of claim 1, wherein said diameter of said first region is sufficient to create a gap between the wall of a nested filter well (20) and the wall of a corresponding receiver well (21).

## Patentansprüche

1. Eine Multiwell-Vorrichtung mit einer Filterplatte mit mehreren Filterwells (20), von denen jedes eine am Boden des Wells befindliche Membran (30) umfasst, und einer Aufnehmerplatte mit mehreren Aufnehmerwells (21), von denen jedes einen Boden besitzt, wobei die Aufnehmerplatte eingerichtet ist, die Filterplatte in einer ineinandergeschachtelten Beziehung aufzunehmen, wodurch sich jedes der Filterwells (20) in ein jeweiliges Aufnehmerwell (21) zu einer von dem Boden des Aufnehmerwells beabstandeten Position erstreckt und **dadurch** in jedem Aufnehmerwell einen ersten Bereich, welcher ein von dem Filterwell eingenommener Bereich ist, und einen zweiten Bereich, welcher ein von einem Filterwell nicht eingenommener Bereich ist, festlegt, wobei der erste Bereich einen Querschnitt besitzt, der größer ist als der des zweiten Bereichs, **dadurch gekennzeichnet, dass** jedes der Aufnehmerwells einen Bereich (32) mit einem nicht gleichmäßigen Querschnitt besitzt, der einen Übergang zwischen dem Querschnitt des ersten Bereichs des Aufnehmerwells (21) und dem Querschnitt des zweiten Bereichs des Aufnehmerwells (21) bildet, wobei das Aufnehmerwell (21) von dem ersten Bereich zu dem zweiten Bereich an einem Punkt an oder unter der Stelle jeder Membran (30) des Filterplattenwells (30) übergeht, wenn sich jedes Filterplattenwell (20) in der ineinandergeschachtelten Beziehung mit einem entsprechenden Aufnehmerplattenwell (21) befindet, und der erste Bereich einen im wesentlichen quadratischen Querschnitt und der zweite Bereich einen im wesentlichen kreisförmigen Querschnitt besitzt.

2. Eine Multiwell-Vorrichtung gemäß Anspruch 1, wobei die Aufnehmerplatte eine Abfasung bzw. Abschrägung (35) besitzt, die entlang dem Umfang der Anordnung der Aufnehmerwells (21) ausgebildet ist.

3. Die Multiwell-Vorrichtung gemäß Anspruch 1, wobei jede Membran (30) eine wirksame Fläche zur Ausführung einer Filtration besitzt und wobei der zweite Bereich jedes Aufnehmerwells (21) so konfiguriert ist, dass Fluid unbehindert von der Fläche jeder Membran (30) zu dem zweiten Bereich gelangen kann.

4. Die Multiwell-Vorrichtung gemäß Anspruch 1, wobei der Durchmesser des ersten Bereichs ausreichend ist, um einen Zwischenraum zwischen der Wand eines ineinandergeschachtelten Filterwells (20) und der Wand eines entsprechenden Aufnehmerwells (21) zu bilden.

## Revendications

1. Dispositif à plusieurs puits comportant une plaque filtrante ayant une pluralité de puits filtrants (20), chacun comprenant une membrane (30) positionnée au niveau du fond du puits, et une plaque réceptrice ayant une pluralité de puits récepteurs (21) ayant chacun un fond, ladite plaque réceptrice étant adaptée pour recevoir ladite plaque filtrante, dans une relation d'emboîtement, de sorte que chacun desdits puits filtrants (20) s'étend dans un puits récepteur respectif (21) vers une position espacée dudit fond du puits récepteur, définissant ainsi dans chacun desdits puits récepteurs une première zone qui est une zone occupée par un puits filtrant, et une seconde zone qui est une zone non occupée par un puits filtrant, ladite première zone ayant une section transversale supérieure à ladite seconde zone, **caractérisé en ce que** chacun desdits puits récepteurs a une zone (32) de section transversale non uniforme fournissant une transition entre la section transversale de ladite première zone de chaque puits récepteur (21) et la section transversale de ladite seconde zone dudit puits récepteur (21), ledit puits récepteur (21) effectuant une transition depuis ladite première zone vers ladite seconde zone au niveau d'un point situé au niveau de l'emplacement de chaque membrane des puits de la plaque filtrante (30), ou sous celui-ci, lorsque chaque puits de la plaque filtrante (20) est dans une relation d'emboîtement avec un puits de la plaque récepteur correspondant (21), et
ladite première zone a une section transversale sensiblement carrée, et ladite seconde zone a une section transversale sensiblement circulaire.

2. Dispositif à plusieurs puits selon la revendication 1, ladite plaque réceptrice ayant un chanfrein (35) formé le long de la périphérie dudit réseau de puits récepteurs (21).

3. Dispositif à plusieurs puits selon la revendication 1, dans lequel chacune desdites membranes (30) a une aire effective pour effectuer une filtration, et dans lequel la seconde zone de chaque puits récepteur (21) est configurée de telle sorte qu'un fluide peut être transféré de manière non obstruée depuis ladite aire de chacune desdites membranes (30) vers chacune desdites secondes zones.

4. Dispositif à plusieurs puits selon la revendication 1, dans lequel ledit diamètre de ladite première zone est suffisant pour créer un espace entre la paroi d'un puits filtrant (20) emboîté et la paroi d'un puits récepteur (21) correspondant.
